# EUROPEAN PATENT SPECIFICATION

(11) **EP 0 638 586 B1**
(45) Date of publication and mention of the grant of the patent: **20.10.1999**
(21) Application number: 94112043.8
(22) Date of filing: 02.08.1994
(51) Int. Cl.: C07H 19/06

(54) **Nucleoside derivatives and methods for producing them**
Nukleosidderivate und Verfahren zu deren Herstellung
Dérivés de nucléosides et leur préparation

(30) Priority: 04.08.1993 JP 19333293; 01.08.1994 JP 18010994
(43) Date of publication of application: 15.02.1995
(73) Proprietor: Ajinomoto Co., Inc., Tokyo 104 (JP)
(72) Inventor: Takamatsu, Satoshi, c/o Central Res. Lab., Kawasaki-ku, Kawasaki-shi, Kanagawa-ken (JP); Shiragami, Hiroshi, c/o Central Res. Lab., Kawasaki-ku, Kawasaki-shi, Kanagawa-ken (JP); Uchida, Yumiko, c/o Central Res. Lab., Kawasaki-ku, Kawasaki-shi, Kanagawa-ken (JP); Izawa, Kunisuke, c/o Central Res. Lab., Kawasaki-ku, Kawasaki-shi, Kanagawa-ken (JP)
(74) Representative: Strehl Schübel-Hopf & Partner

(56) References cited:
- EP-A- 0 280 128
- EP-A- 0 292 101
- EP-A- 0 325 537
- EP-A- 0 470 355
- EP-A- 0 519 464
- DD-A- 279 247
- DE-A- 2 224 380
- US-A- 3 539 550
- US-A- 3 870 700
- US-A- 3 873 516
- US-A- 4 914 233
- CHEMICAL ABSTRACTS, vol. 95, no. 13, 1981, Columbus, Ohio, US; abstract no. 115951, page 750 ; & CS-A-184 526 (CECH) 15 August 1980
- CHEMICAL ABSTRACTS, vol. 85, no. 9, 1976, Columbus, Ohio, US; abstract no. 63304, page 590 ; & JP-A-7 636 467 (TANABE SEIYAKU CO. LTD) 27 March 1976
- CHEMICAL ABSTRACTS, vol. 109, no. 11, 1988, Columbus, Ohio, US; abstract no. 93541, page 767 ; & HU-A-43 615 (MAGYAR TUDOMANYOS AKADEMIA) 30 November 1987

## Description

### Field of the Invention

The present invention provides a novel method for producing 3'-azido-3'-deoxythymidine (azidothymidine, AZT, zidovudine), which is useful as an anti-viral agent, and its related compounds and also provides intermediates for the production thereof.

### Prior Art

It has been reported that 3'-azido-3'-deoxythymidine has a strong anti-viral activity against human immunodeficiency viruses (HIVs) and is noticeably effective for curing acquired immune deficiency syndrome (AIDS) (R. Yarchoan et al., The Lancet, 1 (8481), pp. 575-580, March 15, 1986). At present, therefore, the compound is used for curing AIDS and AIDS-related complications (ARC).

For producing 3'-azido-3'-deoxythymidine on a laboratory scale, there are known two methods. One uses thymidine as a starting material and introduces an azido group into its 3'-position (R. Glinski et al., J. Chem. Soc. D, (15), pp. 915-916, 1970; R. Glinski et al., J. Org. Chem., 38 (25), pp. 4299-4305, 1973); and the other consists in linking the sugar moiety into which an azide group has been introduced to the base moiety of a nucleic acid (N. Dyatkina et al., Bioorg. Khim., 12 (8), pp. 1048-1053, 1986; G. Fleet et al., Tetrahedron Lett., 28 (31), pp. 3615-3618, 1987; G. Fleet et al., Tetrahedron, 44 (2), pp. 625-636, 1988; C. Chu et al., Tetrahedron Lett., 29 (42), pp. 5349-5352, 1988). The former method may be put into industrial application but has the problem of requiring thymidine, which is high-priced and is difficult to obtain on industrial scale, as the starting material. According to the latter method, the production of the sugar moiety into which an azido group has been introduced is difficult and, in addition, both α- and β-isomers are produced when the saccharide moiety is linked to the base moiety of a nucleic acid and these must be separated from each other. For these reasons, it is difficult to say that the latter method is industrially useful.

Several uridine derivatives have already been known-to be useful as antiviral agents or as intermediates in the synthesis of other biologically active nucleosides. Thus US-A-3 870 700 discloses 2-halogen-2-deoxy-5-substituted uridines, wherein the 5-substituent may e.g. be halogen, a thiocyanate or a mercapto group.

Chemical Abstracts, vol. 95, no. 13, 1981, Abstract no. 115951 discloses 2'-halo-2-deoxy-5-fluorouridine and 3,5-di-O-benzoyl derivatives thereof.

DD-A-279 247 discloses a process for producing 1-β-D-allofuranosylthymine nucleosides having a hydroxy substituent in 2-position and a fluorine substituent in 3-position of the furanosyl moiety. The compounds may be produced by partial saponification of 1-(2,3,5-tribenzoyl-6-deoxy-β-D-allofuranosyl)thymine with ammonia to produce the 5-0-benzoyl derivatives.

EP-A-0 280 128 relates to a process for producing 1-(3-azido-2,3-dideoxy-β-D-erythro-pentofuranosyl)pyrimidines by cyclisation of the corresponding 3'-sulphonylated, 5'-protected compound and cleaving the obtained 2,3-anhydro ring by reaction with a metal azide.

EP-A-0 519 464 discloses 2',3'-dideoxy-2',3'-didehydronucleoside derivatives which are useful as antiviral agents. These compounds are produced by reacting a nucleoside derivative, such as 5-methyluridine with an orthoester of an organic acid to produce a 2',3'-O-alkoxyethylidene nucleoside compound, which compound is then reacted with hydrogen bromide in acetic acid or acetyl bromide to produce the corresponding 2'-deoxy-2'-bromo-3',5'-O-diacetyl compound which is subjected to reduction with Zn powder to produce the corresponding olefin.

### Problems to be Solved by the Invention

The object of the present invention is to provide industrially useful methods for producing 3'-azido-3'-deoxythymidine and its related compounds, using other compounds than thymidine, which is high-priced and is difficult to be obtained in large amounts, as starting materials.

### Means for Solving the Problems

The present inventors assiduously studied novel methods for producing 3'-azido-3'-deoxythymidine and its related compounds, using other starting materials than thymidine. As a result, it was found that when 5-methyluridine is used as a starting material, 5'-protected thymidines may be produced which are intermediates which can easily be converted into 3'-azido-3'-deoxythymidine and its related compounds. On the basis of these findings, the present invention has been completed.

First, the present invention provides compounds of the following general formula (1), which are intermediates to be converted into 3'-azido-3'-deoxythymidine and its related compounds. 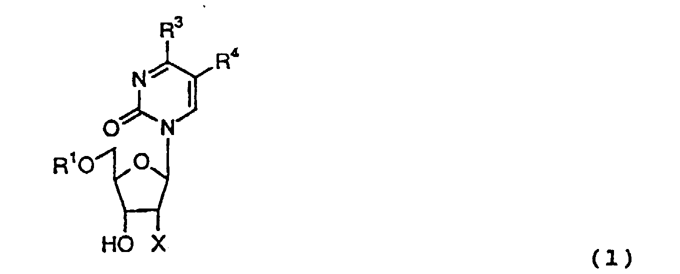 wherein X represents a halogen atom; R¹ represents an acyl group having 1 to 7 carbon atoms; R³ represents an optionally protected hydroxyl group, or an optionally protected amino group; R⁴ represents an alkyl group having 1 to 12 carbon atoms, a hydrogen atom, or a halogen atom.

Secondly, the present invention provides a method for producing compounds of the above-mentioned formula (1), characterized by reacting a compound of a general formula (3): 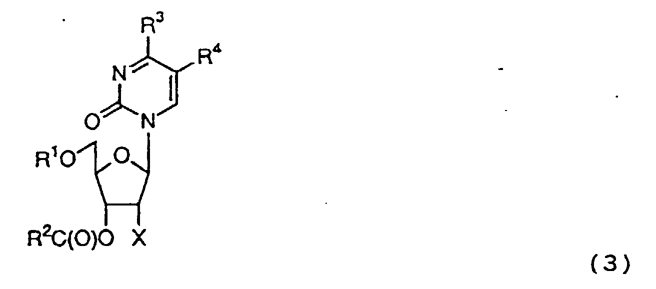 wherein X represents a halogen atom; R¹ represents an acyl group having 1 to 7 carbon atoms,
R² represents an alkyl group having 1 to 6 carbon atoms; R³ represents an optionally protected hydroxyl group, or an optionally protected amino group; R⁴ represents an alkyl group having 1 to 12 carbon atoms, a hydrogen atom or a halogen atom, with hydrazine monohydrate or triethylamine.

Thirdly, the present invention provides a method for producing compounds of the general formula (7) : 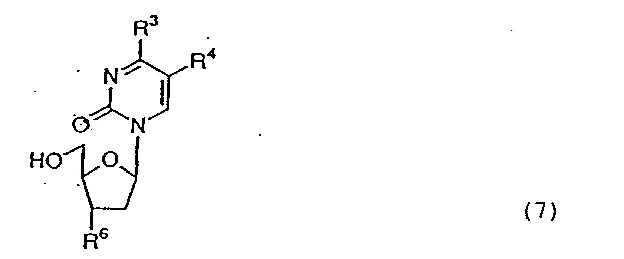 wherein R³ and R⁴ are as defined above, and R⁶ represents an azido group, a cyano group or a fluoro atom, characterized by the steps of:
reducing a compound of the general formula (1): 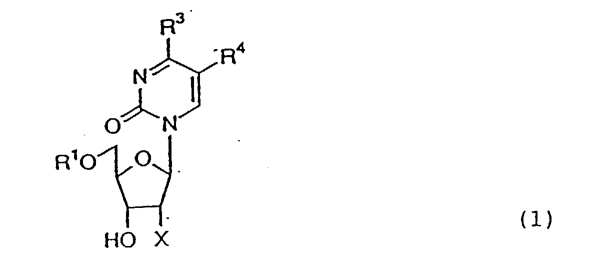 wherein X, R¹, R³ and R⁴ are as defined above, with a palladium catalyst or a nickel catalyst in the presence of a hydrogen donor, to give a compound of the general formula (4) : 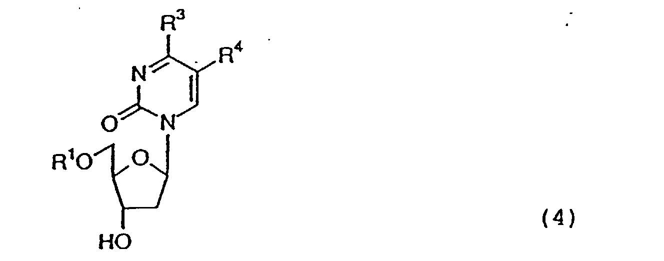 wherein R¹, R³ and R⁴ are as defined above,
then reacting the compound of formula (4) with a sulfonyl halide to give a compound of the general formula (5) : 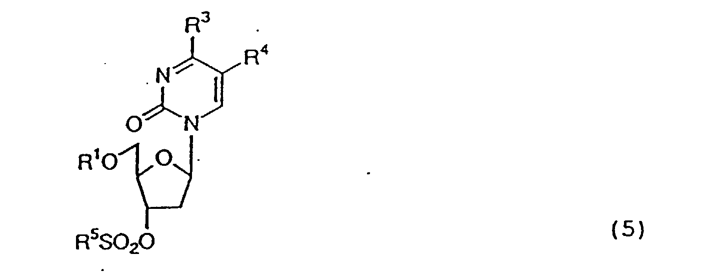 wherein R¹, R³ and R⁴ are as defined above, and R⁵ represents an aryl group, an alkyl group, or a halogenoalkyl group,
then subjecting the compound of formula (5) to ring-closure in the presence of a base to give a compound of the general formula (6) : 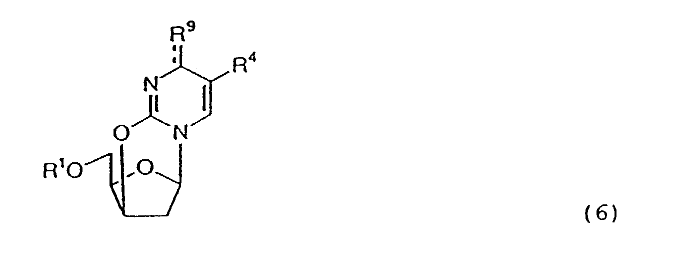 wherein R¹ and R⁴ are as defined above and R⁹ represents an optionally protected oxygen atom, or an optionally protected amino group,
subjecting the compound of formula (6) to ring-cleavage with an azide, a cyanide or a fluoride,
and removing the hydroxyl-protective group R¹ at the 5'-position in the formula by deacylation, the deacylation step being carried out before or after the ring-cleavage.

As one typical example of the above-mentioned compounds of formula (1), mentioned is 2'-deoxy-2'-bromo-5'-O-acetyl-5-methyluridine. This 2'-deoxy-2'-bromo-5'-O-acetyl-5-methyluridine is an important intermediate which may be converted into the above-mentioned 3'-azido-3'-deoxythymidine of formula (7).

Moreover, X in the above-mentioned compounds of formula (1) is selected from halogen atoms. As examples of a halogen atom, mentioned are fluorine, chlorine, bromine, iodine, etc.

R¹ in the above mentioned compounds of formula (1) is an acyl group having 1 to 7 carbon atoms. As examples of an acyl group having 1 to 7 carbon atoms, mentioned are an acetyl group, benzoyl group, etc.

R³ in the above-mentioned compounds of formula (1) is selected from an optionally protected hydroxyl group, or an optionally protected amino group. As examples of an optionally protected hydroxyl group and an optionally protected amino group, mentioned are a hydroxyl group, a trimethylsilyloxy group, a t-butyldimethylsilyloxy group, an acetyloxy group, a benzoyloxy group, a benzyloxy group, an amino group, an acetylamino group, an benzoylamino group, an benzylamino group, etc.

R⁴ in the above-mentioned compounds of formula (1) is selected from an alkyl group having 1 to 12 carbon atoms, hydrogen atom, or a halogen atom. As examples of an alkyl group having 1 to 12 carbon atoms, mentioned are a methyl group, an ethyl group, an isopropyl group, an benzyl group, a vinyl group, an aryl group, an ethynyl group, a 1-propynyl group, a 2-propynyl group, etc.; as examples of a halogen atom, mentioned are fluorine, chlorine, bromine, iodine, etc.

The above-mentioned are not intended to be limitative.

Compounds of the following formula (3) such as typically 2'-deoxy-2'-bromo-3',5'-O-diacetyl-5-methyluridine, which are the starting materials of the present invention, may be produced according to the method disclosed in Japanese Patent Application No. 3-245290. 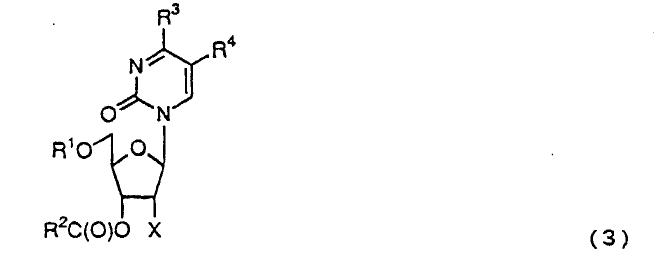 wherein X represents a halogen atom; R¹ represents an acyl group having 1 to 7 carbon atoms, R² represents an alkyl group having 1 to 6 carbon atoms; R³ represents an optionally protected hydroxyl group, or an optionally protected amino group; R⁴ represents an alkyl group having 1 to 12 carbon atoms, a hydrogen atom or a halogen atom.

Precisely, these may be obtained by reacting a compound, such as typically 5-methyluridine, of the general formula (8) : 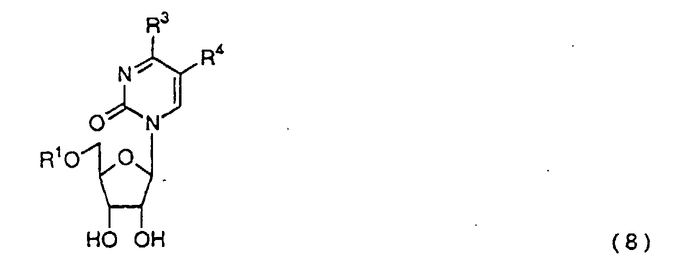 wherein R¹ represents an acyl group having 1 to 7 carbon atoms, R³ represents an optionally protected hydroxyl group, or an optionally protected amino group; R⁴ represents an alkyl group having 1 to 12 carbon atoms, or a halogen atom, with an orthoester of an organic acid, such as a trialkyl orthoacetate (MeC(OR)₃), etc., in acetic acid to convert it into a 2',3'-O-alkoxyalkylidene compound of the general formula (9): 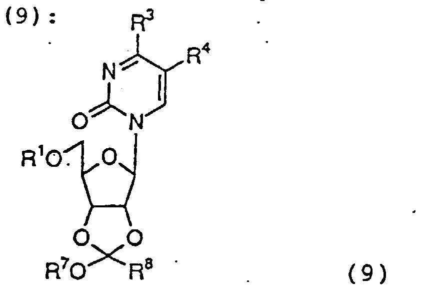 wherein R¹ represents an acyl group having 1 to 7 carbon atoms, R³ represents an optionally protected hydroxyl group, or an optionally protected amino group; R⁴ represents an alkyl group having 1 to 12 carbon atoms, a hydrogen atom or a halogen atom; R⁷ and R⁸ each represent an alkyl group having 1 to 6 carbon atoms, then preferably concentrating the reaction mixture under reduced pressure, before or after the compound formed has been isolated from the mixture, and thereafter reacting it with a solution of a carboxylic acid containing a hydrogen halide, such as a solution of acetic acid containing hydrogen bromide, and/or a carboxylic acid halide such as acetylbromide.

As the weak base to be used for producing the compound of the above-mentioned formula (1) from the compound of the above-mentioned formula (3) especially preferred is hydrazine monohydrate or triethylamine. Buffers that have been adjusted to have suitable pH by suitable methods using the above-mentioned bases may also be used. The amount of the base to be used is suitably from 1 to 10 equivalents relative to the compound to be reacted with the base, but an excessive amount of the base may also be used with no problem. As the reaction solvent, usable are alcohol solvents such as methanol, etc., as well as acetonitrile, water, etc. The reaction system may be or may not be dewatered.

The reaction is effected at temperatures ranging from -40°C to +40°C. The reaction time is not defined, as depending on the reaction temperature and the amount of the base to be used. In general, it is from 0.5 to 120 hours. To isolate the compound formed, the base is neutralized with an acid such as hydrochloric acid, etc. or with an ion-exchanging resin, then the reaction mixture is concentrated, and thereafter the compound formed may be isolated by ordinary extraction using an organic solvent such as ethyl acetate, etc. After the reaction, the reaction mixture may be subjected to the next step for reduction, without isolating the product formed.

The reactions which are mentioned above have made possible the selective removal of the protective group at the 3'-position of the above-mentioned compounds of formula (3).

Therefore, it is possible to easily produce the above-mentioned compounds of formula (1), which are important intermediates to be converted into 3'-azido-3'-deoxythymidine and its related compounds according to the present invention. Furthermore, we can use the above-mentioned compounds of formula (8) including 5-methyluridine, which is low-priced and is easily obtainable in a large amount, as starting materials.

The conversion of the compound of the above-mentioned formula (1) into the compound of the above-mentioned formula (7) according to the present invention comprises reductive removal of the 2'-positioned halogen from the compound of formula (1) to give the compound of formula (4), substitution of the 3'-substituent of the compound of formula (4) by a split-off group such as a sulfonyl group, a halogen atom or the like, conversion of the resulting compound into its 2,3'-anhydride, and introduction of an azido group or the like into its 3'-position by the use of a nucleophilic reagent to give the compound of formula (7) such as typically 3'-azido-3'-deoxythymidine.

As the palladium catalyst and the nickel catalyst to be used for reducing the compound of formula (1) to the compound of formula (4), for example, mentioned are from 5 to 20% palladium-carbon, palladium-barium sulfate, palladium-barium carbonate, and Raney nickel. As the hydrogen donor, usable are cyclohexene, hydrazine, 1,4-cyclohexadiene, formic acid, ammonium formate, hydrogen gas, etc. When hydrogen gas is used, in general, the pressure in the reaction system is somewhat elevated, but ordinary pressure may also be employed with no problem. The reaction may be effected in ordinary solvents. For instance, usable are alcohols such as methanol, etc. as well as acetonitrile, water, dimethylformamide, etc., and mixed solvents of these. The reaction may be effected in the presence of a salt. As usable salts, for example, mentioned are sodium acetate, ammonium acetate, ammonium formate, sodium carbonate, etc.

The reaction is effected, in general, at temperatures falling within the range of from -40°C to +60°C, for 1 to 48 hours. After the reaction has finished, the catalyst used is filtered from the reaction mixture and the reaction mixture is concentrated to be directly used in the next reaction step, without isolating the product formed therefrom. If, however, the product is desired to be isolated from the reaction mixture, any ordinary method may be employed. For instance, the catalyst is taken out from the reaction mixture by filtration, the reaction mixture is then concentrated, the resulting residue is dissolved in water, and the aqueous solution thus formed is extracted with an organic solvent such as ethyl acetate, etc.

If desired, the compound of formula (1) is produced from the compound of formula (3) and is directly subjected to the present reduction without isolating it. For instance, an acid such as formic acid or acetic acid is added to the base that was used in the previous reaction step to form a salt, and a palladium catalyst or a nickel catalyst is added to the reaction system to effect the intended reduction to give the compound of formula (4).

To convert the compound of formula (4) into the compound of formula (5), the former is reacted with a sulfonyl halide in the presence of a basic catalyst such as pyridine, etc. in a basic solvent. To isolate the compound of formula (5) thus formed, the base is neutralized with an acid such as hydrochloric acid, etc. or with an ion-exchanging resin, if a basic catalyst was used, then the reaction mixture is concentrated and thereafter it is subjected to ordinary extraction with an organic solvent such as ethyl acetate, etc. to isolate the intended compound. After the reaction, if desired, the reaction mixture may be directly subjected to the next reaction without isolating the product therefrom.

To convert the compound of formula (5) into the compound of formula (6), the former is subjected to ring-closure in the presence of a base. As the base to be used for the ring-closure, for example, mentioned are sodium hydroxide, potassium hydroxide, ammonia, sodium methylate, potassium phthalimide, etc. The amount of the base to be used may be selected from the range of from 1 to 10 molar equivalents relative to the compound of formula (5). The reaction temperature for the ring-closure may be selected from the range of from -40°C to +60°C. After the reaction, the compound of formula (6) may be isolated by treating the reaction mixture by an ordinary method.

To convert the compound of formula (6) into the compound of formula (7), the former is subjected to ring-cleavage with an azide, a cyanide or a fluoride. As the azide to be used for the reaction, for example, mentioned are alkali metal azides such as sodium azide, lithium azide, etc., as well as ammonium azide, trimethylsilyl azide, etc. As the cyanide to be used for the reaction, for example, mentioned are alkali metal cyanides such as sodium cyanide, lithium cyanide, etc. As the fluoride to be used for the reaction, for example, mentioned are hydrogen fluoride, lithium fluoride, potassium fluoride, tetrabutylammonium fluoride, diethylaminosulfato trifluoride, etc. The reaction is effected in a solvent capable of dissolving both the compound of formula (6) and the azide, cyanide or fluoride. As the solvent suitable for the reaction, for example, mentioned are dimethylformamide, dimethylsulfoxide, etc.

The protective group in 5'-position may be removed at any desired stage for the conversion of the compound of formula (5) to the compound of formula (7), by a suitable method such as saponification optionally using an alkali. The compound of formula (7), such as typically 3'-azido-3'-deoxythymidine, may be isolated from the reaction mixture by an ordinary purifying method, for example, by purification with adsorptive resins or by crystallization.

### Examples

Next, the present invention will be explained in more detail by means of the following examples.

### Referential Example 1:

### Production of 2'-deoxy-2'-bromo-3',5'-O-diacetyl-5-methyluridine from 5-methyluridine:

42.5 g (350 mmol) of trimethyl orthoacetate were added to 125 ml of a slurry of acetic acid containing 75.3 g (250 mmol) of 85.7%-purity 5-methyluridine and reacted at 50°C for one hour. The reaction mixture was concentrated under reduced pressure to obtain 2',3'-O-methoxyethylidene-5-methyluridine. To the thus-obtained 2',3'-O-methoxyethylidene-5'-methyluridine, added were 207 ml of acetonitrile. The resulting solution was heated up to 50°C, and a mixture comprising 202 g (3 equivalents) of 30% hydrogen bromide/acetic acid and 30.8 g (one equivalent) of acetic acid bromide was dropwise added thereto over a period of 2 hours. The reaction mixture was heated up to 60°C and reacted for further 4 hours. This was cooled to 10°C, and 125 ml of water were added thereto. Then, this was neutralized with aqueous 25% sodium hydroxide solution, and the acetonitrile layer was separated. The aqueous layer was extracted with acetonitrile. The organic layers were combined and concentrated under reduced pressure. The resulting residue was recrystallized from methanol to obtain 71.5 g (176 mmol) of 2'-deoxy-2'-bromo-3',5'-O-diacetyl-5-methyluridine. Its yield was 70.6%, based on 5-methyluridine.
NMR Analysis (¹H, CDCl₃):
δ 1.95 (3H, s, 5 Me), 2.16 (3H, s, 5' OAc ), 2. 19 (3Hs, 3' OAc) , 4.39 (3H, m, 5' H + 4' H), 4. 54 (1H, dd, j = 6.0, 6.0 Hz, 2' H), 5.18 (1H, dd, j = 6, 0, 3.7Hz, 3' H), 6.23 (1H, d, J = 6.0 Hz, 1 ' H), 7.19 (1H, q, J = 1.8 Hz, 6H), 8.70 (1H, br, 3 NH).

### Example 1:

### Production of 2'-deoxy-2'-bromo-5'-O-acetyl-5-methyluridine from 2'-deoxy-2'-bromo-3',5'-O-diacetyl-5-methyluridine:

400 ml of acetonitrile and 2.16 g (3 equivalents) of water were added to 16.2 g (40.0 mmol) of 2'-deoxy-2'-bromo-3',5'-O-diacetyl-5-methyluridine, and stirred. The resulting solution was cooled to 0°C, 6.01 g (3 equivalents) of hydrazine monohydrate were added thereto and reacted for 20 hours at the temperature. 100 ml of water were added to the reaction mixture, which was then neutralized to pH of 6.8 by adding 6 N-hydrochloric acid thereto. The acetonitrile layer was separated, washed with saturated saline solution, dried with anhydrous magnesium sulfate and then concentrated under reduced pressure to obtain 13.5 g of 2'-deoxy-2'-bromo-5'-O-acetyl-5-methyluridine. Its purity was 81.8%. Its yield was 76.0%.
NMR Analysis (¹H, CDCl₃):
δ 1.95 (3H, s, 5Me), 4.22 (1H, t, J = 5, 0Hz, 4 'H), 4.32-4.46 (3H, m, 5' H + 3 ' H), 4.53 (1H, t, j = 5.4Hz, 2'H), 6.18 (1H, d, J = 5.4Hz, 1'H) , 7.26(1H, bs, 6H), 9.05 (1H, bs, 3NH)
NMR Analysis (¹³C, CDCl₃):
δ 12.71, 20.90, 43.89, 63.19, 70.28, 81.6 7, 90.43, 111.64, 116.52, 135.10. 170.36
IR Spectral Analysis (KBr):
cm⁻¹ 3434, 3075, 1670, 1471, 1385, 1272, 12 36, 1092, 1042, 784, 565, 490
UV Spectral Analysis (H₂O):
λ^{max-} 261.6 nm
Mass Spectral Analysis (FAB Mode):

| | |
|---|---|
| calculated (M⁺H⁺, C₁₂H₁₆O₆N₂Br) | 363.0192 |
| measured | 363.0193 |

### Example 2:

### Production of 2'-deoxy-2'-bromo-5'-O-acetyl-5-methyluridine from 2'-deoxy-2'-bromo-3',5'-O-diacetyl-5-methyluridine:

10 ml of acetonitrile were added to 405 mg (1.0 mmol) of 2'-deoxy-2'-bromo-3',5'-O-diacetyl-5-methyluridine, and stirred. The resulting solution was cooled to 0 deg., 100 mg (2 equivalents) of hydrazine monohydrate were added thereto and reacted for 17.8 hours at the temperature. The reaction solution was subjected to HPLC analysis, which showed the production of 84.1 % of 2'-deoxy-2'-bromo-5'-O-acetyl-5-methyluridine, based on the HPLC area.

### Example 3:

### Production of 2'-deoxy-2'-bromo-5'-O-acetyl-5-methyluridine from 2'-deoxy-2'-bromo-3',5'-O-diacetyl-5-methyluridine:

10 ml of acetonitrile were added to 405 mg (1.0 mmol) of 2'-deoxy-2'-bromo-3',5'-O-diacetyl-5-methyluridine, and stirred. The resulting solution was cooled to 0 deg., 100 mg (2 equivalents) of hydrazine monohydrate and 90 mg (5 equivalents) of water were added thereto and reacted for 18.3 hours at the temperature. The reaction solution was subjected to HPLC analysis, which showed the production of 84.9% of 2'-deoxy-2'-bromo-5'-O-acetyl-5-methyluridine, based on the HPLC area.

### Example 4:

### Production of 2'-deoxy-2'-bromo-5'-O-acetyl-5-methyluridine from 2'-deoxy-2'-bromo-3',5'-O-diacetyl-5-methyluridine:

10 ml of acetonitrile were added to 405 mg (1.0 mmol) of 2'-deoxy-2'-bromo-3',5'-O-diacetyl-5-methyluridine, and stirred. The resulting solution was cooled to 0 deg., 150 mg (3 equivalents) of hydrazine monohydrate and 90 mg (5 equivalents) of water were added thereto and reacted for 2.1 hours at the temperature. The reaction solution was subjected to HPLC analysis, which showed the production of 71.3% of 2'-deoxy-2'-bromo-5'-O-acetyl-5-methyluridine, based on the HPLC area.

### Example 5:

### Production of 2'-deoxy-2'-bromo-5'-O-acetyl-5-methyluridine from 2'-deoxy-2'-bromo-3',5'-O-diacetyl-5-methyluridine:

20 ml of methanol were added to 810 mg (2.0 mmol) of 2'-deoxy-2'-bromo-3',5'-O-diacetyl-5-methyluridine, and stirred. The resulting solution was cooled to 0 deg., 405 mg (2 equivalents) of triethylamine were added thereto, and the reaction solution was warmed up to room temperature and reacted for 5 days at the temperature. The reaction solution was subjected to HPLC analysis, which showed the production of 96.3% of 2'-deoxy-2'-bromo-5'-O-acetyl-5-methyluridine, based on the HPLC area.

### Example 6:

### Production of 5'-O-acetylthymidine from 2'-deoxy-2'-bromo-5'-O-acetyl-5-methyluridine:

12.47 g (28.1 mmol) of 2'-deoxy-2'-bromo-5'-O-acetyl-5-methyluridine were dissolved in 240 ml of methanol, and 6.91 g (3 equivalents) of sodium acetate were added thereto. To the reaction mixture, added were 1.25 g of 5% palladium-barium sulfate catalyst (0.1 time). Then, the reaction system was filled with hydrogen gas and reacted for 24 hours at room temperature. The catalyst was removed from the reaction mixture, 100 ml of water were added, and the reaction mixture was neutralized to have pH of 7.0 by adding aqueous 25% sodium hydroxide solution thereto. The resulting solution was concentrated to about 100 ml, sodium chloride was added thereto until saturation, and the thus-saturated solution was extracted with ethyl acetate. The thus-extracted organic layer was washed with saturated saline solution, dried with anhydrous magnesium sulfate and then concentrated under reduced pressure to obtain white crystals. The crystals were washed with a small amount of methylene chloride to obtain 3.04 g (10.7 mmol) of 5'-O-acetylthymidine. Its yield was 38.1%.
NMR Analysis (¹H, CDCl₃):
δ 1.94 (3H, d, J = 1.4 H z, 5Me), 2.18 (1H, m, 2'Hα), 2.43 (1H, ddd, J=4.5, 6.2, 13. 5Hz, 2' Hβ). 4.13 (1H, q, J = 4.5 H z. 4'H) , 4.30 (1H, d d, J = 3.5, 12.3, 5'Hα), 4.39 (1H, m, 3'H), 4.40 (1H, dd, J=4.5, 12.3 H z, 5' Hβ) , 6.27 (1H, t, J = 6.5 Hz, 1' H), 7.27 (1H, q, J =1. 4 Hz, 6H), 8. 21 (1 H, bs, 3NH)

### Example 7:

### Production of 5'-O-acetylthymidine from 2'-deoxy-2' -bromo-3',5'-O-diacetyl-5-methyluridine:

20.26 g (50.0 mmol) of 2'-deoxy-2'-bromo-3',5'-O-diacetyl-5-methyluridine were dissolved in 500 ml of acetonitrile. This solution was cooled to 0°C, and 5.01 g (2 equivalents) of hydrazine monohydrate were added thereto to start the reaction therebetween. After 11 hours, 9.21 g (4 equivalents) of formic acid, 3.34 g of 10% palladium-carbon catalyst (water content of 60.7%; 0.1 weight equivalent) and 250 ml of methanol were added thereto, and the reaction system was filled with hydrogen gas to again start the reaction at room temperature. After 14 hours, the catalyst was removed from the reaction mixture by filtration, and the reaction liquid was concentrated under reduced pressure. The resulting residue was dissolved in water and extracted with ethyl acetate, and the thus-extracted organic layer was washed with saturated saline solution. The organic layer was dried with anhydrous magnesium sulfate and then concentrated under reduced pressure to obtain 27.3 g of 5'-O-acetylthymidine. The product was used in the next reaction, without being purified.

### Example 8:

### Production of 5'-O-acetyl-3'-O-methanesulfonylthymidine from 5'-O-acetylthymidine:

The 5'-O-acetylthymidine obtained in Example 3 was dissolved in 100 ml of pyridine and cooled to 0°C. Then, 11.57 g (2 equivalents) of methanesulfonyl chloride were added thereto. After these were reacted at 0°C for 1.5 hours, 5.79 g (one equivalent) of methanesulfonyl chloride were added thereto and reacted at 0°C for 1.5 hours. The reaction mixture was concentrated under reduced pressure, the residue was dissolved in methylene chloride, and the insoluble substances were removed by filtration. The organic layer was washed with water, aqueous, saturated sodium hydrogencarbonate solution, water and saturated saline solution in this order, dried with anhydrous sodium sulfate and concentrated under reduced pressure to obtain 10.4 g of 5'-O-acetyl-3'-O-methanesulfonylthymidine. The product was used in the next reaction, without being isolated.
NMR Analysis (¹H, CDCl₃):
δ1.94 (3H, d, J=1.1Hz, 5Me), 2.13 (3H, s, 5'OAc), 2.33 (1H, ddd, J=6.9, 8.3, 14.7Hz, 2'Hα), 2.68 (1H, ddd, J=2.9, 6.0, 14.7Hz, 2'Hβ), 3.12 (3H, s, 3'OMs), 4.37 (2H, m, 5'H), 4.44 (1H, m, 4'H), 5.27 (1H, ddd, J=2.9, 5.7, 6.9Hz, 3'H), 6.26 (1H, dd, J=6.0, 8.3Hz, 1'H), 7.20 (1H, d, J=1.1Hz, 6H), 8.85 (1H, bs, 3NH)

### Example 9:

### Production of 3'-azido-3'-deoxythymidine from 5'-O-acetyl-3'-O-methanesulfonylthymidine:

100 ml of ethanol were added to 10.4 g of 5'-O-acetyl-3'-O-methanesulfonylthymidine obtained in the previous step and heated under reflux. To the resulting solution, dropwise added was a solution prepared by dissolving 1.15 g (one equivalent) of sodium hydroxide in 10 ml of water, over a period of 45 minutes. This was heated for further 45 minutes, then cooled to room temperature and concentrated under reduced pressure. The resulting residue was dissolved in 50 ml of dimethylformamide, 2.11 g (1.5 equivalents) of lithium azide were added thereto and reacted at 120°C for 16.5 hours. The reaction solution was cooled to room temperature and subjected to HPLC analysis, which showed the production of 3.21 g (12.0 mmol) of azidothymidine. Its yield was 24.0%, based on 2'-deoxy-2'-bromo-3',5'-O-diacetyl-5-methyluridine.

### Advantages of the Invention

As is obvious from the above-mentioned description, useful nucleoside derivatives, such as AZT, and their intermediates may be produced from low-priced starting materials such as 5-methyluridine, according to the present invention. Therefore, the present invention is extremely useful in industrial application.

## Claims

1. Compounds of the general formula (1) : 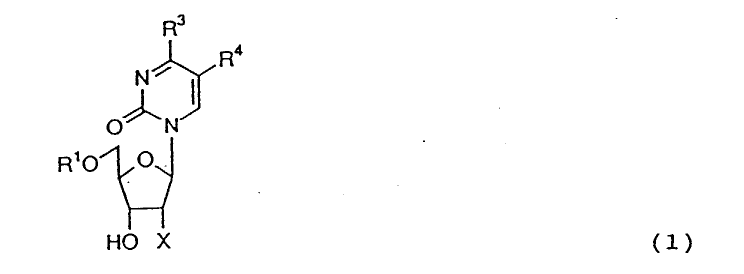 wherein X represents a halogen atom; R¹ represents an acyl group having 1 to 7 carbon atoms; R³ represents an optionally protected hydroxyl group, or an optionally protected amino group; R⁴ represents an alkyl group having 1 to 12 carbon atoms, a hydrogen atom, or a halogen atom.

2. A compound according to claim 1 represented by the following formula (2) :

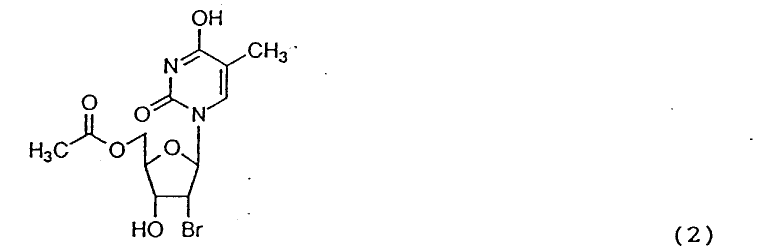

3. A method for producing compounds of formula (1) : 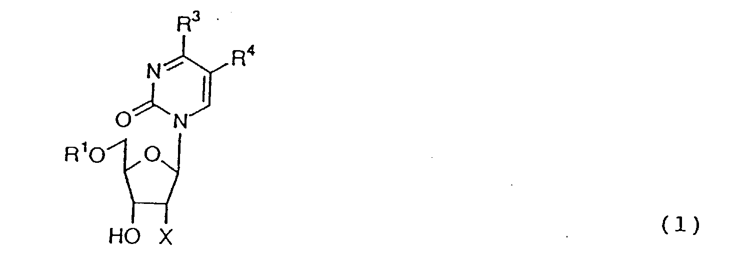 wherein X, R¹, R³ and R⁴ are as defined in claim 1, characterized by reacting a compound of the general formula (3) : 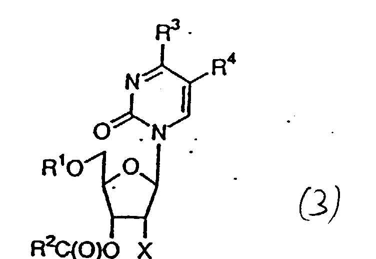 wherein X, R¹, R³, and R⁴ are as defined above, and R² represents an alkyl group having 1 to 6 carbon atoms, with hydrazine monohydrate, or triethylamine.

4. A method for producing compounds of the general formula (7) : 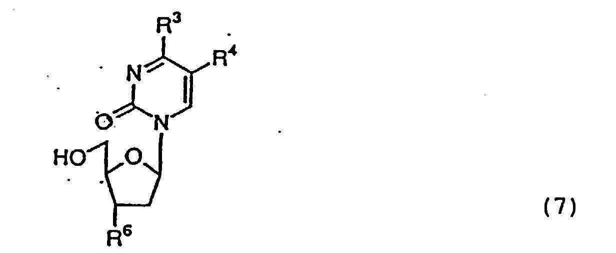 wherein R³ and R⁴ are as defined in claim 1, and R⁶ represents an azido group, a cyano group or a fluoro atom, characterized by the steps of:
reducing a compound of the general formula (1): 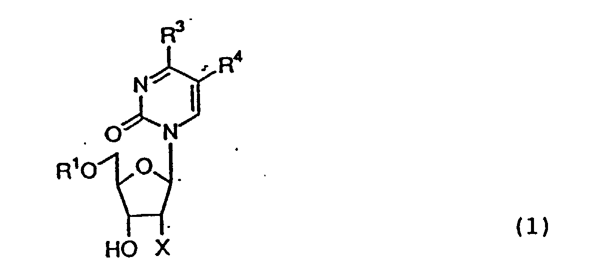 wherein X, R¹, R³ and R⁴ are as defined in claim 1, with a palladium catalyst or a nickel catalyst in the presence of a hydrogen donor, to give a compound of the general formula (4) : 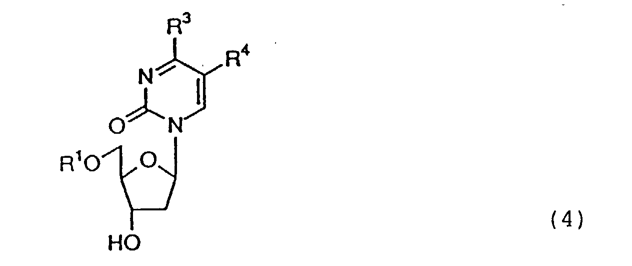 wherein R¹, R³ and R⁴ are as defined in claim 1,
then reacting the compound of formula (4) with a sulfonyl halide to give a compound of the general formula (5) : 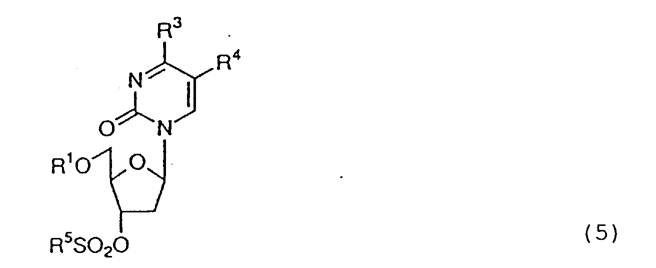 wherein R¹, R³ and R⁴ are as defined in claim 1, and R⁵ represents an aryl group, an alkyl group, or a halogenoalkyl group,
then subjecting the compound of formula (5) to ring-closure in the presence of a base to give a compound of the general formula (6) : 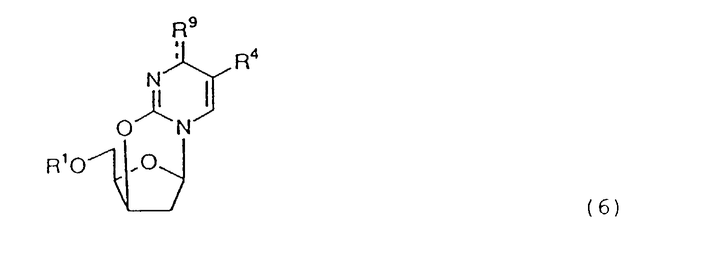 wherein R¹, and R⁴ are as defined in claim 1 and R⁹ represents an optionally protected oxygen atom, or an optionally protected amino group,
subjecting the compound of formula (6) to ring-cleavage with an azide, a cyanide or a fluoride,
and removing the hydroxyl-protective group R¹ at the 5'-position in the formula by deacylation, the deacylation step being carried out before or after the ring-cleavage.

## Patentansprüche

1. Verbindungen der allgemeinen Formel (1) : 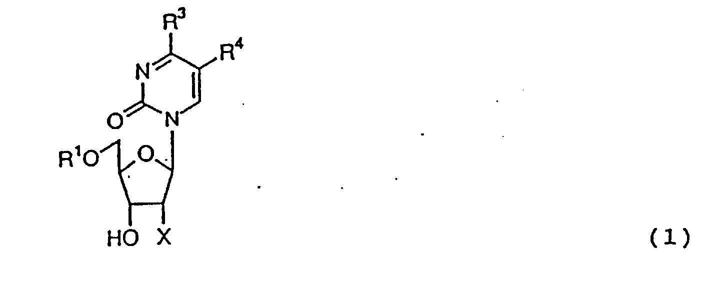 worin X ein Halogenatom darstellt, R¹ eine Acylgruppe mit 1 bis 7 Kohlenstoffatomen darstellt, R³ eine gegebenenfalls geschützte Hydroxygruppe oder eine gegebenenfalls geschützte Aminogruppe darstellt, R⁴ eine Alkylgruppe mit 1 bis 12 Kohlenstoffatomen, ein Wasserstoffatom oder ein Halogenatom darstellt.

2. Verbindung gemäß Anspruch 1, die durch die folgende Formel (2) dargestellt wird:

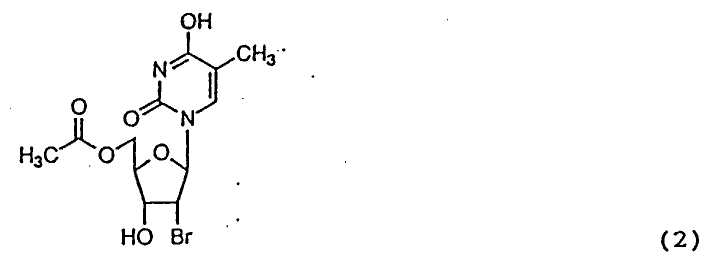

3. Verfahren zur Herstellung von Verbindungen der Formel (1): 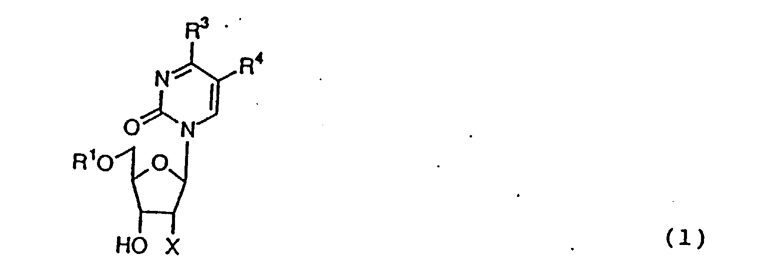 worin X, R¹, R³ und R⁴ wie in Anspruch 1 definiert sind, dadurch gekennzeichnet, daß eine Verbindung der allgemeinen Formel (3) 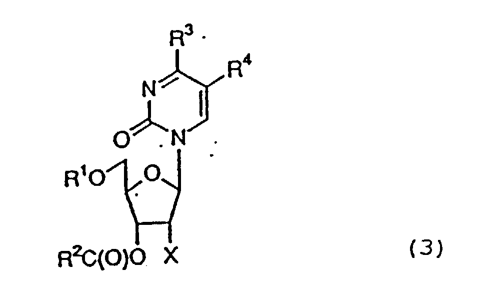 worin X, R¹, R³ und R⁴ wie vorstehend definiert sind und R² eine Alkylgruppe mit 1 bis 6 Kohlenstoffatomen darstellt, mit Hydrazinmonohydrat oder Triethylamin umgesetzt wird.

4. Verfahren zur Herstellung von Verbindungen der allgemeinen Formel (7): 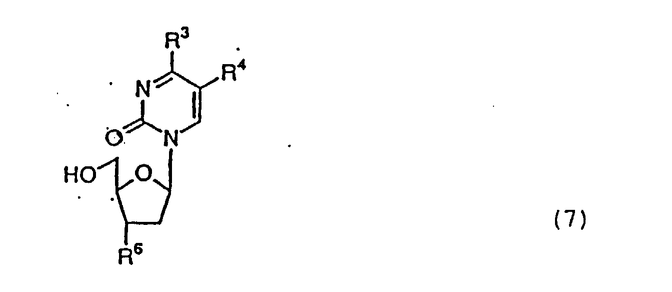 worin R³ und R⁴ wie in Anspruch 1 definiert sind und R⁶ eine Azidogruppe, eine Cyangruppe oder ein Fluoratom darstellt, gekennzeichnet durch die folgenden Stufen:
Reduzieren einer Verbindung der allgemeinen Formel (1): 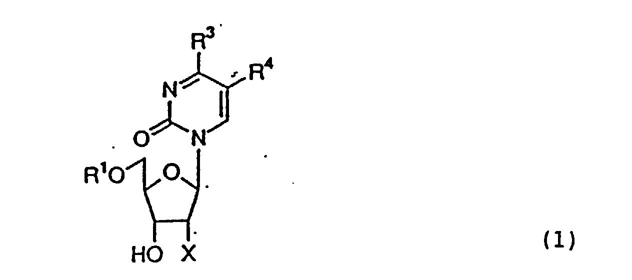 worin X, R¹, R³ und R⁴ wie in Anspruch 1 definiert sind, mit einem Palladiumkatalysator oder einem Nickelkatalysator in Anwesenheit eines Wasserstoffdonors, wobei eine Verbindung der allgemeinen Formel (4) erhalten wird: 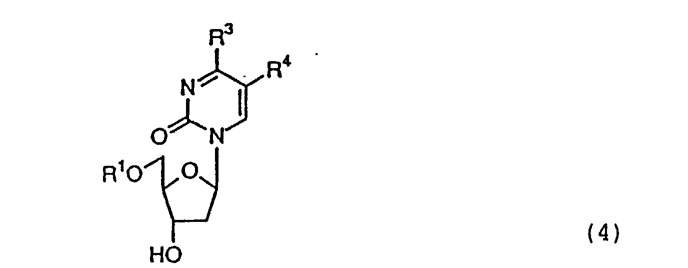 worin R¹, R³ und R⁴ wie in Anspruch 1 definiert sind,
anschließendes Umsetzen der Verbindung der Formel (4) mit einem Sulfonylhalogenid, wobei eine Verbindung der allgemeinen Formel (5) erhalten wird: 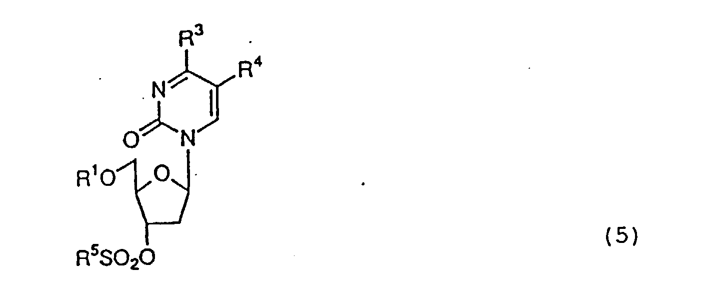 worin R¹, R³ und R⁴ wie in Anspruch 1 definiert sind und R⁵ eine Arylgruppe, eine Alkylgruppe oder eine Halogenalkylgruppe darstellt,
anschließendes Unterziehen der Verbindung der Formel (5) einem Ringschluß in Anwesenheit einer Base, wobei eine Verbindung der allgemeinen Formel (6) erhalten wird: 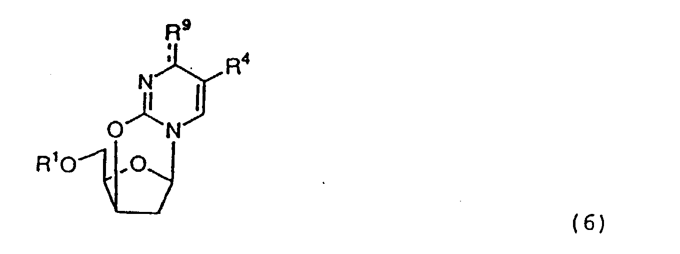 worin R¹ und R⁴ wie in Anspruch 1 definiert sind und R⁹ ein gegebenenfalls geschütztes Sauerstoffatom oder eine gegebenenfalls geschützte Aminogruppe darstellt,
Unterziehen der Verbindung der Formel (6) einer Ringspaltung mit einem Azid, einem Cyanid oder einem Fluorid, und
Entfernen der Schutzgruppe R¹ für Hydroxygruppen an der 5'-Position in der Formel durch Deacylierung, wobei die Stufe der Deacylierung vor oder nach der Ringspaltung durchgeführt wird.

## Revendications

1. Composés de formule générale (1) : 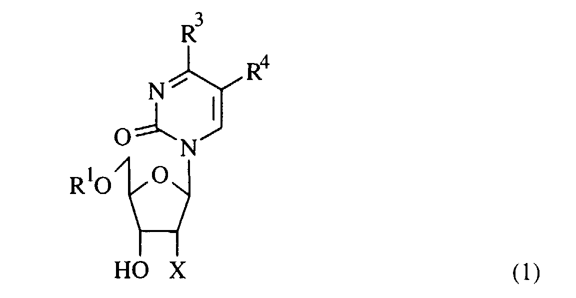 où X représente un atome d'halogène ; R¹ représente un groupe acyle ayant 1 à 7 atomes de carbone et R³ représente un groupe hydroxyle éventuellement protégé ou un groupe amino éventuellement protégé ; R⁴ représente un groupe alkyle ayant 1 à 12 atomes de carbone, un atome d'hydrogène ou un atome d'halogène.

2. Composé selon la revendication 1 représenté par la formule générale (2) :

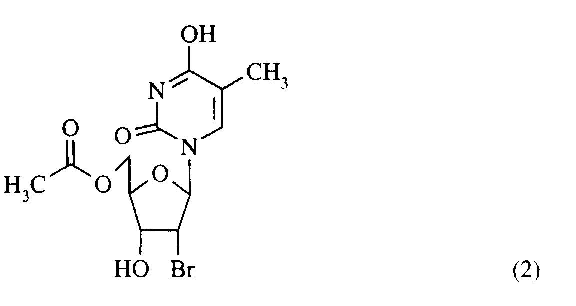

3. Méthode pour la production des composés de formule (1) : 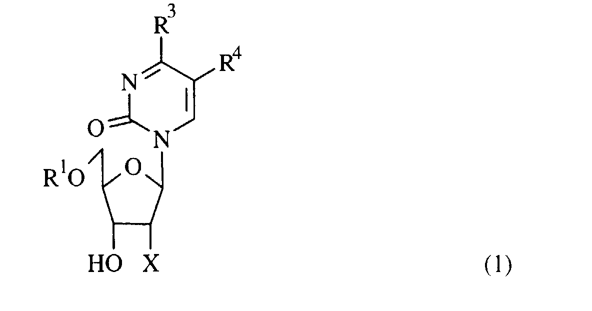 où X, R¹, R³ et R⁴ sont tels que définis dans la revendication 1, caractérisée en ce que l'on fait réagir un composé de formule générale (3) : 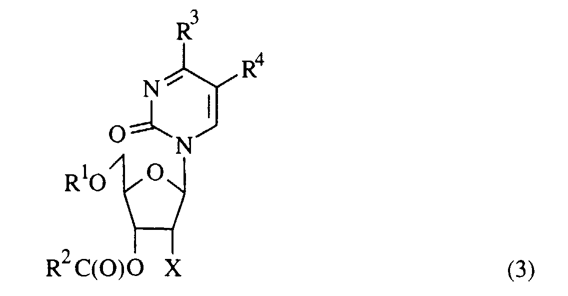 où X, R¹, R³ et R⁴ sont tels que définis ci-dessus et R² représente un groupe alkyle ayant 1 à 6 atomes de carbone, avec le monohydrate d'hydrazine ou de la triéthylamine.

4. Méthode pour la production des composés de formule générale (7) : 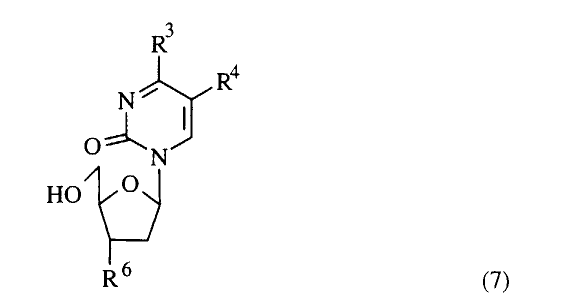 où R³ et R⁴ sont tels que définis dans la revendication 1 et R⁶ représente un groupe azido, un groupe cyano ou un atome de fluor, caractérisée par les étapes :
de la réduction d'un composé de formule générale (1) : 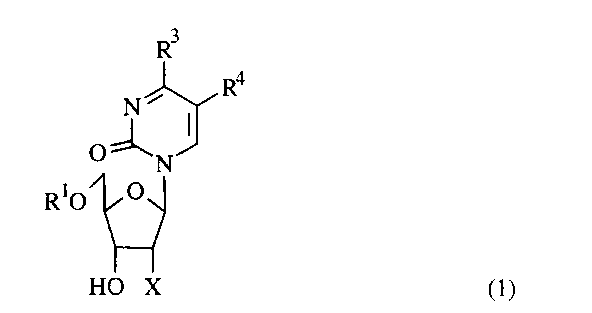 où X, R¹, R³ et R⁴ sont tels que définis dans la revendication 1, avec un catalyseur au palladium ou un catalyseur au nickel en présence d'un donneur d'hydrogène, pour obtenir un composé de formule générale (4) : 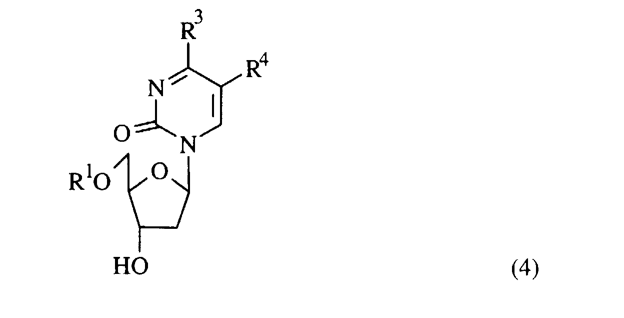 où R¹, R³ et R⁴ sont tels que définis dans la revendication 1,
puis de la réaction du composé de formule (4) avec un halogénure de sulfonyle pour obtenir un composé de formule générale (5) : 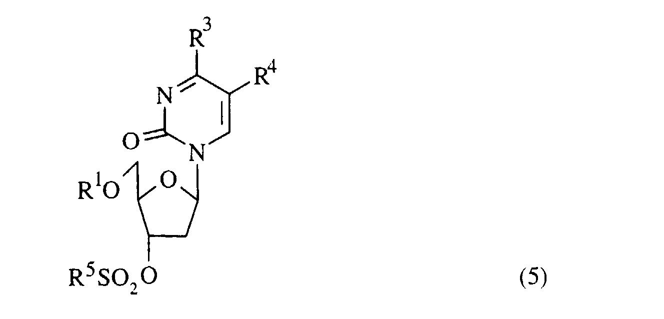 où R¹, R³ et R⁴ sont tels que définis dans la revendication 1 et R⁵ représente un groupe aryle, un groupe alkyle ou un groupe halogénoalkyle,
puis de la soumission du composé de formule (5) à une réaction de cyclisation en présence d'une base pour obtenir un composé de formule générale (6) : 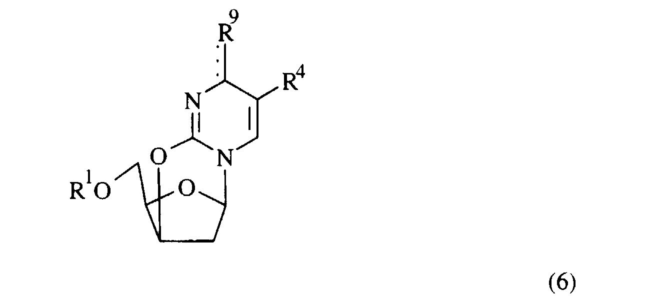 où R¹ et R⁴ sont tels que définis dans la revendication 1 et R⁹ représente un atome d'oxygène éventuellement protégé ou un groupe amino éventuellement protégé,
de la soumission du composé de formule (6) à une réaction d'ouverture de cycle au moyen d'un azoture, d'un cyanure ou d'un fluorure,
et de l'élimination du groupe protecteur d'hydroxyle R' en position 5' dans la formule par désacylation, l'étape de désacylation étant réalisée avant ou après l'ouverture du cycle.
